(19) Europäisches Patentamt
European Patent Office
Office européen des brevets

(11) **EP 3 207 917 A1**

(12) **EUROPEAN PATENT APPLICATION**

(43) Date of publication:
**23.08.2017 Bulletin 2017/34**

(51) Int Cl.:
*A61K 8/02* (2006.01)        *A61K 8/19* (2006.01)
*C09C 1/40* (2006.01)        *C09C 1/30* (2006.01)

(21) Application number: **16305203.8**

(22) Date of filing: **22.02.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Imerys Minerals Limited**
**Par**
**Cornwall PL24 2SQ (GB)**

(72) Inventors:
• **Jolliff, Sam**
  **Cornwall PL14 8LQ (GB)**
• **Wang, Bo**
  **Union City, CA 94587 (US)**

(74) Representative: **Johnston, Magnus George**
**Haseltine Lake LLP**
**Redcliff Quay**
**120 Redcliff Street**
**Bristol BS1 6HU (GB)**

(54) **VISUALLY ENHANCED PERLITE PARTICULATE**

(57)    Perlite microspheres having a modified visual appearance, methods of making the perlite microspheres, a composition, such as a personal care, cosmetic or cleaning composition, comprising the perlite microspheres, a visually enhanced exfoliating agent comprising the perlite microspheres, and to the use of the perlite microspheres in personal care, cosmetic or cleaning compositions.

**FIGURE 1**

EP 3 207 917 A1

**Description**

**TECHNICAL FIELD**

The present invention is directed to perlite microspheres having a modified visual appearance, to methods of making the perlite microspheres, to a composition, such as a personal care, cosmetic or cleaning composition, comprising the perlite microspheres, a visually enhanced exfoliating agent comprising the perlite microspheres, and to the use of the perlite microspheres in personal care, cosmetic or cleaning compositions.

**BACKGROUND OF THE INVENTION**

It is known to add particulate matter to skin cleansing compositions, such as shower gels and bath gels, in order to impart a scrub feel and to aid exfoliation of the uppermost layer of skin upon lathering. More recently, there has been a growing trend to incorporate plastic micro-beads into skin cleansing compositions. However, plastic micro-beads have been found to enter the water course and end up in lakes, seas and oceans. This has led to some environmental groups to call for a ban on plastic micro-beads. Thus, there is ongoing need to develop new and even improved particulates for use in shower gels and the like, which do not suffer from the purported environmental drawbacks of plastic micro-beads and/or provide an enhanced experience for the user.

**SUMMARY OF THE INVENTION**

According to a first aspect, the present invention is directed to a perlite microsphere having a modified visual appearance, for example, an enhanced visual appearance; and optionally

(a) a $-\Delta b^*$ or $-\Delta b$ compared to the unmodified perlite microsphere; or
(b) a $+\Delta b^*$ or $+\Delta b$ compared to the unmodified perlite microsphere; or
(c) a $-\Delta a^*$ or $-\Delta a$ compared to the unmodified perlite microsphere; or
(d) a $+\Delta a^*$ or $+\Delta a$ compared to the unmodified perlite microsphere;
(e) a $b^*$ or $b$ which is further from 0 than the unmodified perlite microsphere; or
(f) an $a^*$ or $a$ which is further from 0 than the unmodified perlite microsphere; or
(g) a colour density which is greater than the colour density of the unmodified perlite microsphere.

According to a second aspect, the present invention is directed to a method of making a perlite microsphere according to the first aspect, comprising treating a perlite microsphere such that its visual appearance is modified compared to the unmodified perlite microsphere prior to treatment.
According to a third aspect, the present invention is directed to a composition comprising perlite microspheres according to the first aspect.
According to a fourth aspect, the present invention is directed to a visually enhanced exfoliating agent comprising, or consisting essentially of, or consisting of, perlite microspheres according to the first aspect.
According to a fifth aspect, the present invention is directed to the use of perlite microspheres according to the first aspect in a personal care, cosmetic or cleaning composition to visually modify the appearance, for example, to enhance user-appeal, of the composition.
According to a sixth aspect, the present invention is directed to the use of perlite microspheres according to the first aspect as a visually enhanced exfoliating component in a personal care, cosmetic or cleaning composition.
According to a seventh aspect, the present invention is directed to the use of perlite microspheres according to the first aspect in: (i) a personal care composition, for example, a personal care cleansing composition such as, for example, a gel, for example, a shower gel, optionally wherein the perlite microspheres provide a scrub feel, skin exfoliation, or both; or (ii) a hair shampoo, for example, an anti-dandruff shampoo, optionally wherein the perlite microspheres aid or provide exfoliation of the skin of the scalp.

**BRIEF DESCRIPTION OF THE DRAWINGS**

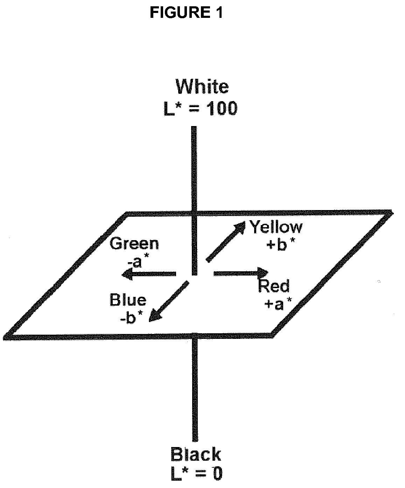

Figure 1 is a diagram representing CIELAB color space.

**DETAILED DESCRIPTION OF THE INVENTION**

The term "modified visual appearance" used herein refers to a perlite microsphere having an optical appearance or property which, to the human eye, is different to the optical appearance of the microsphere in its unmodified form, e.g., natural form. This includes, for example, a microsphere having a modified colour, gloss, shine, matte, sparkle, lustre, fluorescence, transparency, opacity or mirror-effect. For the avoidance of doubt, the term "modified visual appearance" is not referring to a modification in the morphology or surface topology, e.g., texture, of the perlite microsphere, nor is it referring to properties which are not perceptible by the unaided human eye.

The term "enhanced visual appearance" used herein refers to a microsphere having enhanced user-appeal, for example, increased visibility (e.g., contrast) to the user/consumer when the microsphere is present in a composition, for example, a personal care, cosmetic or cleaning composition, owing to its modified visual appearance. Increased visibility could be due to the microsphere having, for example, a modified colour, gloss, shine, matte, sparkle, lustre, fluorescence, transparency, opacity or mirror-effect, which contrasts more strongly (i.e., relative to a unmodified perlite microsphere) with the composition in which it is incorporated. An "enhanced visual appearance" may also increase the detectability of the perlite microspheres.

Additionally, the perlite microspheres provide a scrub feel and/or exfoliating properties when included in a personal care composition, for example a personal care cleansing composition comprising a gel, as a partial or total replacement for plastic micro-beads currently used in shower and bath gels. In certain embodiments, the perlite microspheres are mildly abrasive. The mechanical action of the application provides an initial scrub feel, then aids exfoliation of the upper layer of skin. In certain embodiments described herein, the perlite microspheres are substantially hollow. In such embodiments, following the initial scrub feel, continued mechanical action breaks down the hollow spheres or microspheres further enhancing exfoliation of the upper layer of the skin.

The perlite microspheres may provide gentle scrubbing action properties when included in a cleaning composition. In certain embodiments, continued mechanical action may break down the hollow microspheres further enhancing the gentle scrubbing action of the cleaning composition.

It has now been surprisingly found that perlite microspheres can be modified in order to produce perlite microspheres having a modified, e.g., non-natural, visual appearance, for example, to produce a perlite microsphere which is coloured or more intensely coloured than the unmodified material. The use of such perlite microspheres may enhance the user-appeal of products and compositions into which they are incorporated. For example, in certain embodiments, perlite microspheres are coloured and, for example, used as abrasive particles in personal care, cleaning or other compositions. In such compositions the perlite particles may be broken down upon application by the user, and, thus, the use of coloured perlite microspheres may aid the user to more easily or clearly perceive the mechanical action of the abrasive particles breaking down upon use, thereby further enhancing the user's experience.

*Modified visual appearance*

In the following sections, references to "a perlite microsphere" includes references to "perlite microspheres".

The perlite microsphere has a modified, e.g., non-natural, visual appearance, as described above. In certain embodiments, the perlite microsphere has an enhanced visual appearance, as described above. The modified or enhanced visual appearance may be characterized in terms of an optical property, such as colour, i.e., the perlite microsphere has a colour which is modified compared to the perlite microsphere before modification. In other embodiments, the perlite microsphere following modification may have, or may have a modified, gloss, shine, matte, sparkle, lustre (e.g., radiance, gloss and/or brilliance, fluorescence, transparency, opacity or mirror-effect).

In certain embodiments the perlite microsphere of the present invention may have a non-natural visual appearance due to the optical properties of the perlite microsphere, as listed above, resulting from the perlite microsphere being coloured.

In certain advantageous embodiments, the perlite microsphere has a modified colour or is coloured with respect to the unmodified perlite microsphere. In certain embodiments, the colour of the perlite microsphere following modification is characterised in terms of a colour space, for example, Hunter Lab colour space (L, a, b, +/-$\Delta$L, +/-$\Delta$a, +/-$\Delta$b, $\Delta$E) or CIELAB colour space (L*, a*, b*, +/-$\Delta$L*, +/-$\Delta$a*, +/-$\Delta$b*, $\Delta$E*), or in terms of colour density. Further details of the Hunter Lab and CIELAB colour spaces are appended to the detailed description.

In certain embodiments, the perlite microsphere, following modification, has:

(a) a -$\Delta$b* or -$\Delta$b compared to the unmodified perlite microsphere; or
(b) a +$\Delta$b* or +$\Delta$b compared to the unmodified perlite microsphere; or
(c) a -$\Delta$a* or -$\Delta$a compared to the unmodified perlite microsphere; or
(d) a +$\Delta$a* or +$\Delta$a compared to the unmodified perlite microsphere;
(e) a b* or b which is further from 0 than the unmodified perlite microsphere; or
(f) an a* or a which is further from 0 than the unmodified perlite microsphere; or
(g) a colour density which is greater than the colour density of the unmodified perlite microsphere.

In certain embodiments, a +$\Delta$a* or +$\Delta$a means that the perlite microsphere, following modification, tends to be or is redder than the unmodified form. In certain embodiments, a $-\Delta a^*$ or $-\Delta a$ means that the perlite microsphere, following modification, tends to be or is greener than the unmodified form. In certain embodiments, a $-\Delta b^*$ or $-\Delta b$ means that the perlite microsphere, following modification, tends to be or is bluer than the unmodified form. In certain embodiments, a $+\Delta b^*$ or $+\Delta b$ means that the perlite microsphere, following modification, tends to be or is yellower than the unmodified form.

In certain embodiments, $-\Delta b^*$ or $-\Delta b$ is at least -5, i.e., $b^*$ or b of the modified perlite is at least 5 units lower than the $b^*$ or b of the unmodified perlite, for example, $-\Delta b^*$ or $-\Delta b$ is at least -10, or at least -15, or at least -20.

In certain embodiments, $+\Delta b^*$ or $+\Delta b$ is at least 2, i.e., $b^*$ or b of the modified perlite is at least 2 units higher than the $b^*$ or b of the unmodified perlite, for example, $+\Delta b^*$ or $+\Delta b$ is at least 3, or at least 4, or at least 5, or at least 6, or at least 7, or at least 8.

Additionally or alternatively, in certain embodiments, $-\Delta a^*$ or $-\Delta a$ is at least -0.5, i.e., $a^*$ or a of the modified perlite is at least 0.5 units lower than the $a^*$ or a of the unmodified perlite, for example, at least 0.75, or at least 1.0, or at least 1.25, or at least about 1.5, or at least 1.75, or at least 2.0, or at least 2.25, or at least 2.5, or at least 2.75, or at least 3.0.

Additionally or alternatively, in certain embodiments, $+\Delta a^*$ or $+\Delta a$ is at least 0.5, i.e., $a^*$ or a of the modified perlite is at least 0.5 units higher than the $a^*$ or a of the unmodified perlite, for example, at least 1.0, or at least 1.5, or at least 2.0, or at least 2.5, or at least 3.0, or at least 3.5, or at least 4.0, or at least 4.5, or at least 5.0, or at least 5.5, or at least 6.0, or at least 6.5, or at least 7.0, or at least 7.5, or at least 8.0, or at least 8.5, or at least 9.0, or at least 9.5, or at least 10.0.

In certain embodiments, $b^*$ or b is at least 2 units further from 0 than the unmodified perlite microsphere, for example, at least 4 units, or at least 6 units, or at least 8 units, or at least 10 units, or at least 12 units, or at least 14 units, or at least 16 units, or at least 18 units, or at least 20 units further from 0 than the unmodified perlite microsphere. In such embodiments, $b^*$ or b is further from 0 in a positive direction or further from 0 in a negative direction.

Additionally or alternatively, in certain embodiments, $a^*$ or a is at least 0.5 units further from 0 than the unmodified perlite microsphere, for example, at least 1.0 units, or at least 2.0 units, or at least 2.5 units, or at least 3.0 units, or at least 3.5 units, or at least 4.0 units, or at least 4.5 units, or at least 5.0 units, or at least 5.5 units, or at least 6.0 units, or at least 6.5 units, or at least 7.0 units, or at least 7.5 units, or at least 8.0 units, or at least 8.5 units, or at least 9.0 units, or at least 9.5 units, or at least 10.0 units further from 0 than the unmodified perlite microsphere. In such embodiments, $a^*$ or a is further from 0 in a positive direction or further from 0 in a negative direction.

In certain embodiments, the modified perlite microsphere has a $b^*$ or b of less than 0, for example, -1 or less, -2 or less, -3 or less, -4 or less, or -5 or less, -7 or less, -9 or less, or -10 or less, or -15 or less, or -20 or less, or -25 or less. In certain embodiments, $b^*$ or b is no less than -25, for example, no less than about -22, or no less than -20.

In certain embodiments, the modified perlite microsphere has a $b^*$ or b of up to about 2, for example, up to about 4, or up to about 6, or p to about 8, or up to about 10, or up to about, or up to about 12, or up to about 14, or up to about 16, or up to about 18, or up to about 20. In certain embodiments, $b^*$ or b is no greater than about 20, for example, no greater than about 15.

In certain embodiments, the modified perlite microsphere an $a^*$ or a of less than 0, for example, -0.5 or less, -1 or less, -2 or less, or -3 or less, or -4 or less, or -5 or less, or -6 or less, or -7 of less, or -8 or less, or -9 or less, or -10 or less. In certain embodiments, $a^*$ or a is no less than about -20, for example, no less than about -15, or no less than about -12, or no less than about -10.

In certain embodiments, the perlite microsphere an $a^*$ or a of greater than 0, for example, at least 1, or at least 2, or at least 4, or at least 5, or at least 6, or at least 7, or at least 8, or at least 9, or at least 10. In certain embodiments, $a^*$ or a is no greater than about 15, for example, no greater than about 12, or no greater than about 10.

In certain embodiments $b^*$ or b is -5 or less, for example -10 or less, or -15 or less, or -20 or less, and $a^*$ or a is no greater than about 10, for example, no greater than about 8, or no greater than about 6, or no greater than about 4, or no greater than about 2, or no greater than about 0, or less than 0, for example, no less than about -3, or no less than about -2.

In certain embodiments $a^*$ or a is up to about 5, for example, up to about 3, or up to about 1, or less than about 0, or -1, or less, -3 or less, or -5 or less, or -7 or less, or -10 or less, and $b^*$ or b is no greater than about 25, for example, no greater than about 20, or no greater than about 15, or no greater than about 13, or no greater than about 10, or no greater than about 8, or no greater than about 6, or no greater than about 4 or no greater than about 2 or no greater than about 0.

In certain embodiments $a^*$ or a, and $b^*$ or b, are both positive, and $a^*$ or a is greater than $b^*$ or b. In certain embodiments $a^*$ or a, and $b^*$ or b, are both positive, and $b^*$ or b is greater than $a^*$ or a. In certain embodiments $a^*$ or a, and $b^*$ or b, are both negative, and $a^*$ or a is greater than $b^*$ or b. In certain embodiments $a^*$ or a, and $b^*$ or b, are both negative integers, and $b^*$ or b is greater than $a^*$ or a.

In certain embodiments $a^*$ or a is negative and $b^*$ or b is positive. In said embodiment, $a^*$ or a may have a value of less than -1, for example, less than -2, or less than -3, or less than -4, or less than -5, or less than -6, or less than -7, or less than -10, or less than -15, and $b^*$ or b may have a value of greater than 1, for example, greater than 2, or greater than 3, or greater than 4, or greater than 5, or greater than 7, or greater than 10, or greater than 15.

In certain embodiments, a* or a is positive and b* or b is negative. In said embodiment, b* or b may have a value of -1 or less, for example, -2 or less, or -3 or less, or -4 or less, or -5 or less, or -6 or less, -7 or less, or -10 or less, or -15 or less, and a* or a may have a value of greater than 1, for example, greater than 2, or greater than 3, or greater than 4, or greater than 5, or greater than 7, or greater than 10, or greater than 15.

In certain embodiments, the sum of: (i) the amount by which a* or a differs from 0, and (ii) the amount by which b* or b differs from 0, is greater for the perlite microsphere which has been modified compared to the unmodified perlite microsphere. In certain embodiments, the sum of (i) and (ii) for the modified perlite microsphere is at least 4.80, for example, at least about 5, or at least about 7.5, or at least about 10, or at least about 15, or at least about 20, or at least about 25, or at least about 30.

In certain embodiments, the modified perlite microsphere as an a* or a which is positive and further from 0 compared to the unmodified perlite microsphere, for example, greater than about 5, and (i) b* or b is less than a* or a, and which may be closer to 0 compared to the unmodified perlite microsphere, or (ii) b* or b is positive and within 1 unit of a* or a.

In certain embodiments, the modified perlite microsphere has a b* or b which is -10 or less, and (i) a* or a is less than 0, for example, greater than about -5, for example, greater than about -2, or (ii) a* or a is no greater than about 4, for example, no greater than about 3, and may always be positive.

In certain embodiments, a* or a is less than 0, for example, -2 or less, optionally wherein b* or b is no greater than about 15 and optionally always positive.

In certain embodiments, a* or a, and b* or b, are both positive integers, and (i) a* or a is greater than b* or b , or (ii) b* or b is greater than a* or a .

In certain embodiments, the modified perlite microsphere has a -ΔL* or -ΔL compared to the unmodified perlite microsphere; and/or L* or L is less than the L* or L of the unmodified perlite microsphere; and/or L* or L is from about 20 to about 70, for example, from about 30 to about 65, or less than about 50.

In certain embodiments the modified perlite microsphere has an L* or L value of greater than about 20, for example, greater than about 25, or greater than about 30, or greater than about 35, or greater than about 40, or greater than about 45, or greater than about 50, or greater than about 55, or greater than about 60, or greater than about 70.

In certain embodiments, the modified perlite microsphere has an L* or L value of less than about 70, for example, less than about 65, or less than about 60, or less than about 55, or less than about 50, or less than about 45, or less than about 40, or less than about 35, or less than about 30.

In certain embodiments, modified perlite microsphere has an L* or L value lower than the L* or L value of the unmodified perlite microsphere. In certain embodiments, the perlite microsphere has an L* or L value higher than the L* or L value of the unmodified perlite microsphere.

In certain embodiments, the modified perlite microsphere has an L* or L of from about 20 to about 70, for example, from about 30 to about 65, or from about 35 to about 60, or from about 40 to about 60, or less than about 50. In certain embodiments the perlite microsphere has an L:* or L of from about 45 to about 60, for example, from about 50 to about 60, or from about 50 to about 55, or from about 55 to about 60.

In certain embodiments, the perlite microsphere has a colour selected from any one or more of the following: yellow, orange, red, violet, blue, indigo, violet, purple, teal, pink, green, cyan, turquoise, lilac, gold, silver, bronze. In certain embodiments, the perlite microsphere is any colour selected from any one or more of the following: yellow, orange, red, violet, blue, indigo, violet, purple, teal, pink, green, and combinations thereof. In certain embodiments, the perlite microsphere has a colour selected from any one of the following: yellow, yellow-green, green, blue-green, blue, blue-violet, violet, violet-red, red, red-orange, orange and orange-yellow.

In certain embodiments, the perlite microsphere has an increased colourfulness relative to an unmodified perlite microsphere. Colourfulness is the visual sensation according to which the perceived colour of an area appears to be more or less chromatic. In certain embodiments, the perlite microsphere has an increased chroma relative to an unmodified perlite microsphere. Chroma refers to the colourfulness relative to the brightness of a similarly illuminated area that appears to be white or highly transmitting. In certain embodiments, the perlite microsphere has an increased saturation relative to an unmodified perlite microsphere. Saturation refers to the colourfulness of a colour relative to its own brightness.

In certain embodiments, the modified perlite microsphere has a modified, for example, enhanced resistance to degradation upon exposure to UV radiation. The ΔE value may be an indicator of UV-induced degradation. In certain embodiments, therefore, the ΔE of the modified perlite microsphere is no greater than 2.5 following exposure to UV radiation for 24 hours, and/or no greater than 10 following exposure to UV radiation for over 750 hours, for example, no greater than 8, or no greater than 5. UV degradation may be determined in accordance with the method described in the Examples section below.

In certain embodiments, the non-optical properties (e.g., hardness, crush strength, and the like) of the modified perlite microsphere are substantially the same as the unmodified perlite microsphere, e.g., the perlite microsphere has about the same hardness and/or crush strength compared to an unmodified perlite microsphere. In certain embodiments, therefore modification in visual appearance does not materially affect other non-visual, non-optical properties of the perlite microsphere.

*Colourant*

In certain embodiments, the modified perlite microsphere is coloured with a colourant

The term "colourant" as used herein refers to any chemical species capable of imparting colour when applied to the perlite microsphere.

In certain embodiments said colourant is a surface treatment, for example, a coating, or coating layer, which may or may not be a continuous coating.

In certain embodiments the colourant comprises, consists essentially of, or consists of at least one colourant selected from: a pigment, a dye and a colouring agent other than a pigment or dye. In certain embodiments the colourant comprises, consists essentially of, or consists of a pigment. In certain embodiments the colourant comprises, consists essentially of, or consists of a dye. In certain embodiments the colourant comprises, consists essentially of, or consists of a colouring agent other than a pigment or dye, for example, a coloured chemical, paint, varnish, emulsion or ink. The colourant may comprise, consist essentially of, or consist of any species capable of being a colourant. In certain embodiments the colourant may comprise, consistent essentially of, or consist of more than one pigment, a dye or a colouring agent other than a pigment or dye. In certain embodiments the colourant comprises, consists essentially of, or consists only of a single pigment, dye or colouring agent other than a pigment or dye.

In certain embodiments the colourant is water-based or water-soluble, for example, in a wet-spray form. In certain embodiments the colourant is not a water-based colourant. In certain embodiments the colourant is not water-soluble. In certain embodiments, the colourant is solvent-based. In certain embodiments the colourant comprises a bonding agent, i.e., an agent, for example, linker which increase adhesion of the colourant to the surface of the perlite microsphere. In certain embodiments the colourant is an organic colourant.

In certain embodiments the colourant is a pigment. In certain embodiments the pigment is an inorganic pigment or an organic pigment. In certain embodiments the pigment is an inorganic pigment, for example, comprising titanium dioxide, zinc oxide, calcium carbonate or fumed silica. The pigment may be in a powder or granular form, or may be suspended or dispersed in a liquid carrier, for example, water or other solvent. In certain embodiments, the pigment is an organic pigment, for example, comprising diazo pigment, a monoazo pigment, a phthalocyanine pigment, or a polycyclic pigment. In certain embodiments, the pigment is a pigment selected from any one of the following: a fluorescent pigment, an ultramarine pigment, metallic pigment, for example a metallic oxide pigment, and any other suitable pigment known in the art.

In certain embodiments the pigment is an ultramarine blue pigment. In certain embodiments the pigment is a copper phthalocyanine green pigment. In certain embodiments the pigment is a copper phthalocyanine blue pigment.

In certain embodiments the pigment comprises, consists essentially of, or is a biological pigment. A biological pigment, also known as a biochrome, is a substance produced by a living organisms that has a colour resulting from selective colour absorption.

In certain embodiments the colourant is a dye. In certain embodiments the dye is selected from any one of the following: an acid dye, a basic dye, an azo dye, a sulfur dye, a reactive dye and a disperse dye. In certain embodiments, the dye is a natural dye.

In certain embodiments the dye is an azo dye. In certain embodiments the azo dye is lithol rubine BK.

In certain embodiments, the colourant comprises, consists essentially of, or consists of at least one metal, for example, a transition metal or a metal selected from any of the following: Ag, Pb, Fe, Cr, Co, Ca, Na and Cu. In certain embodiments the colourant comprises, consists essentially of, or consists of a transition metal, for example, V, Cr, Mn, Fe, Co, Ni, Cu, or Ag. In certain embodiments the colourant comprises, consists essentially of, or consists of a metal selected from any of the following: Ag, Pb, Fe, Cr, Co, Ca, Na or Cu. In certain embodiments the colourant comprises, consists essentially of, or consists of any metal suitable for use in a colourant. In certain embodiments the colourant comprises, consists essentially of, or consists of a metal selected from any of the following: Ag, Pb, Fe, Cr, Co or Cu. In certain embodiments the metal is Ag, or the metal is Pb, or the metal is Fe, or the metal is Cr, or the metal is Ca, or the metal is Na, or the metal is Cu, or the metal is Co. In certain embodiments, the colourant does not comprise a metal silicate.

In certain embodiments, the metal is part of a metal compound, for example, oxide or salt. In certain embodiments, the copper compound is copper phthalocyanine. In certain embodiments, the calcium compound is lithol rubine BK. In certain embodiments, the iron compound is an iron oxide. In certain embodiments the cobalt compound is a cobalt halide, for example, cobalt chloride. In certain embodiments, the aluminium or silicate compound is an aluminiumsilicate. In certain embodiments the sodium, aluminium or silicate compound is sodium aluminiumsilicate. In certain embodiments, the chromium compound is chromium hydroxide.

In certain embodiments the colourant is added in an amount of least about 0.1 wt. % based on the weight of the perlite microsphere starting material which is to be modified, for example, in an amount of at least about 0.2 wt. %, or at least about 0.3 wt. %, or at least about 0.4 wt. %, or at least about 0.5 wt. %, or at least about 0.6 wt. %, or at least about 0.8 wt. %, or at least about 1 wt. %, or at least about 2 wt. %, or at least about 3 wt. %, or at least about 5 wt. %. In certain embodiments the colourant is added in an amount of less than about 25 wt. % based on the weight of the perlite microsphere starting material which is to be modified, for example less than about 20 wt. %, or less than about 15 wt. %, or less than about 10 wt. %, or less than about 5.0 wt. %, or less than about 4.0 wt. %, or less than about 3.0 wt. %, or less than about 2.0 wt. In certain embodiments the colourant is added in an amount of from about 0.1 wt. % to about 10 wt. %, for example, from about 0.1 wt. % to about 7.5 wt. %, or from about 0.1 wt. % to about 5 wt. %, or from about 0.1 wt. % to about 3 wt. %, or from about 0.1 wt. % to about 4 wt. %, or from about 0.1 wt. % to about 2 wt. %, or from about 0.1 wt. % to about 1.0 wt. %.

In certain embodiments the colourant is present as a coating layer about the surface of the perlite microsphere. In certain embodiments the coating layer is adhered to the surface of the perlite microsphere. In certain embodiments the coating layer is adhered to another coating layer which is adhered to the surface of the perlite microsphere, for example, an undercoat. In certain embodiments, the colourant is present as a coating layer that coats the entire surface of the perlite microsphere. In certain embodiments, the colourant is present as a coating layer that coats only a portion of the surface of the perlite microsphere. In certain embodiments, the colourant is present as a coating layer that is not a continuous coating layer. In certain embodiments, the colourant is present as a coating layer that is a continuous coating layer. In certain embodiments, the coating layer is adhered to the surface of the perlite microsphere via a binder. In certain embodiments, the coating layer comprises colourant. In certain embodiments, the coating layer does not comprise a metal silicate.

In certain embodiments the coating layer has a thickness of at least about 50 nm, for example, from about 50 nm to about 5 $\mu$m, for example, from about 50 nm to about 2 $\mu$m, or from about 50 nm to about 1 $\mu$m, or from about 50 nm to about 900 nm. In certain embodiments the coating layer has a thickness of at least about 100 nm, or at about 200 nm, or at least about 300 nm, or at least about 400 nm, or at least about 500 nm, or at least about 600 nm, or at least about 700 nm, or at least about 800 nm.

In certain embodiments, the perlite microsphere comprises a first coating layer (e.g., an undercoat) and a second coating layer on the first coating layer, wherein either or both of the first and second coating layer comprise colourant. In certain embodiments, the second coating layer comprises colourant and the first coating layer does not comprise colourant. In certain embodiments, both the first and second coating layer comprise colourant. In certain embodiments, the first coating layer comprises colourant but the second coating layer does not comprise colourant. In certain embodiments, the first coating layer is of greater thickness, or the same thickness, or of less thickness, than the second coating layer. In certain embodiments, both the first and second coating layer comprise a pigment, dye or other colourant agent other than a pigment or dye. In certain embodiments, only one of the two coating layers comprise a pigment, dye or other colourant agent other than a pigment or dye. In certain embodiments both coating layers comprise a metal. Three, four, or five or more layers are contemplated. In multi-layer embodiments, each layer may have a thickness as described above for a coating layer.

In certain embodiments, the modified perlite microsphere further comprises an undercoat. The undercoat may be comprise, consist essentially of, or consist of any species capable of acting as an undercoat, for example, any species capable of adhering to the surface of the perlite microsphere and having a colourant adhered to the surface of the undercoat. The undercoat may serve to improve adhesion of the colourant to the surface of the perlite microsphere. Different colourants may have different affinities for any particular undercoat.

In certain embodiments this undercoat is an undercoat of silver, biopolymer and/or aminoalkoxysilane. In certain embodiments the undercoat comprises or consists essentially of silver, biopolymer and/or aminoalkoxysilane. In certain embodiments the undercoat is silver. In certain embodiments the undercoat is a biopolymer. In certain embodiments the undercoat is an aminoalkoxysilance.

*Perlite microsphere*

In certain embodiments, the perlite microsphere is an expanded perlite. Typically, expanded perlite includes one or more cells, or parts of cells, in which a cell is a void space partially or entirely surrounded by walls of glass, usually formed from expansion of gases when the glass is in the softened state. Processes for expanding perlite are well known in the art, and include heating perlite in air to a temperature of least about 700 °C, typically between 800 °C and 1100 °C, in an expansion furnace. Exemplary processes for producing expanded perlite are described in US-A-20060075930, the entire contents of which is hereby incorporated by reference. Expanded perlite typically has a bulk volume up to 20 times that of the unexpanded material. In certain embodiments, the perlite microsphere comprises perlite that is not milled, i.e., the perlite is not an expanded milled perlite. In certain embodiments the perlite is milled. In certain embodiments the perlite is milled after it has been expanded.

The perlite microsphere of the present invention may be hollow or solid. In certain embodiments, the perlite microsphere is hollow, for example, substantially closed and hollow. In certain embodiments, the microsphere has a substantially closed cell structure, e.g., sealed cavities normally filled with air. Perlite microspheres can be formed in accordance with the methods described in WO-A-2013053635, the entire contents of which is hereby incorporated by reference. Generally, in this process, perlite ore and propellant is fed into an upright furnace and falls along a drop section through multiple heating zones in a furnace shaft of the furnace. The perlite ore is heated to a critical temperature at which the surfaces of the perlite plasticize and perlite grains are expanded on the basis of the propellant.

Unless otherwise specified, the particle size properties referred to herein for the perlite microspheres are as measured by the well known conventional method employed in the art of laser light scattering, using a CILAS 1064L particle size analyser, as supplied by CILAS (or by other methods which give essentially the same result). In the laser light scattering technique, the size of particles in powders, suspensions and emulsions may be measured using the diffraction of a laser beam, based on an application of Fraunhofer and Mie theory. Such a machine provides measurements and a plot of the cumulative percentage by volume of particles having a size, referred to in the art as the 'equivalent spherical diameter' (e.s.d), less than given e.s.d values. The mean particle size $d_{50}$ is the value determined in this way of the particle e.s.d at which there are 50% by volume of the particles which have an equivalent spherical diameter less than that $d_{50}$ value. The $d_{10}$ value is the value at which 10% by volume of the particles have an e.s.d less than that $d_{10}$ value. The $d_{90}$ value is the value at which 90% by weight of the particles have an e.s.d less than that $d_{90}$ value. The $d_{100}$ value is the value at which 100% by volume of the particles have an e.s.d less than that $d_{100}$ value. The $d_0$ value is the value at which 0% by volume of the particles have an e.s.d less than that $d_0$ value. Thus, the $d_0$ measurement provides a measure of the smallest particles in any given sample (within the limits of measurement of the particle size analyzer).

In certain embodiments, the perlite microsphere has a $d_{10}$ of at least about 10 $\mu$m, for example, at least about 20 $\mu$m, or at least about 30 $\mu$m, or at least about 40 $\mu$m, or at least about 50 $\mu$m, or at least about 75 $\mu$m, or at least about 80 $\mu$m, or at least about 85 $\mu$m, or at least about 90 $\mu$m, or at least about 95 $\mu$m, or at least about 100 $\mu$m.

In certain embodiments, the perlite microsphere has a $d_{10}$ of at least about 30 and a $d_{90}$ of no greater than about 900 $\mu$m, for example, a $d_{10}$ of at least about 30 $\mu$m and a $d_{90}$ of no greater than about 500 $\mu$m, or a $d_{10}$ of at least about 30 $\mu$m and a $d_{90}$ of no greater than about 400 $\mu$m. In certain embodiments the perlite microsphere has a $d_{10}$ of at least about 50 $\mu$m and a $d_{90}$ of no greater than about 900 $\mu$m, for example, a $d_{10}$ of at least about 50 $\mu$m and a $d_{90}$ of no greater than about 500 $\mu$m, or a $d_{10}$ of at least about 50 $\mu$m and a $d_{90}$ of no greater than about 475 $\mu$m, or a $d_{10}$ of at least about 75 $\mu$m and a $d_{90}$ of no greater than about 475 $\mu$m, or a $d_{10}$ of at least about 90 $\mu$m and a $d_{90}$ of no greater than about 475 $\mu$m, or a $d_{10}$ of at least about 90 $\mu$m and a $d_{90}$ of no greater than about 450 $\mu$m. In said embodiments, the perlite microsphere may have a $d_{50}$ of from about 0.1 $\mu$m to about 400 $\mu$m, for example, from about 0.1 $\mu$m to about 200 $\mu$m, or from about 50 $\mu$m to about 200 $\mu$m, or from about 150 $\mu$m to about 400 $\mu$m, or from about 150 $\mu$m to about 350 $\mu$m, or from about 150 $\mu$m to about 250 $\mu$m, or from about 150 $\mu$m to about 200 $\mu$m, or from about 175 $\mu$m to about 300 $\mu$m, or from about 175 $\mu$m to about 250 $\mu$m, or from about 200 $\mu$m to about 300 $\mu$m, or from about 200 um to about 275 $\mu$m, or from about 225 $\mu$m to about 275 $\mu$m, or from about 250 $\mu$m to about 350 $\mu$m, or from about 275 $\mu$m to about 325 $\mu$m, or from about 25 $\mu$m to about 350 $\mu$m, for example, from about 50 $\mu$m to about 350 $\mu$m, or from about 100 $\mu$m to about 350 $\mu$m, or from about 25 $\mu$m to about 100 $\mu$m, or from about 30 $\mu$m to about 80 $\mu$m, or from about 50 $\mu$m to about 100 $\mu$m, or from about 50 $\mu$m to about 75 $\mu$m.

In certain embodiments, the perlite microsphere has a $d_{10}$ of at least about 10 $\mu$m and a $d_{90}$ of no greater than about 150 $\mu$m, for example, a $d_{10}$ of at least about 15 $\mu$m and a $d_{90}$ of no greater than about 135 $\mu$m, or a $d_{10}$ of at least about 20 $\mu$m and a $d_{90}$ of no greater than about 115 $\mu$m, or a $d_{10}$ of at least about 20 $\mu$m and a $d_{90}$ of no greater than about 100 $\mu$m. In such embodiments, the perlite microsphere may have a density of from about 0.20 to about 2.50 g/cc, for example, from about 1.00 to about 2.25 g.cc, or from about 1.50 to about 2.25 g/cc, or from about 1.75 to about 2.25 g/cc, or from about 1.80 to about 2.10 g/cc, or from about 1.90 to about 2.00 g/cc.

In certain embodiments the perlite microsphere has a $d_{10}$ of at least about 50 $\mu$m and a $d_{90}$ of no greater than about 900 $\mu$m, for example, a $d_{10}$ of at least about 50 $\mu$m and a $d_{90}$ of no greater than about 500 $\mu$m, or a $d_{10}$ of at least about 50 $\mu$m and a $d_{90}$ of no greater than about 450 $\mu$m. In such embodiments, the perlite microsphere may have a density of from about 0.20 to about 0.75 g/cc, for example, from about 0.20 to about 0.50 g/cc. Said perlite microsphere may have a $d_{50}$ of from about 150 $\mu$m to about 400 $\mu$m, of from about 150 $\mu$m to about 350 $\mu$m, for example, from about 150 $\mu$m to about 300 $\mu$m, or from about 200 $\mu$m to about 300 $\mu$m, or from about 225 $\mu$m to about 275 $\mu$m, or from about 240 $\mu$m to about 270 $\mu$m.

In certain embodiments, the perlite microsphere has a $d_{10}$ of at least about 50 $\mu$m and a $d_{90}$ of no greater than about 450 $\mu$m, for example, a $d_{10}$ of at least about 70 $\mu$m and a $d_{90}$ of no greater than about 420 $\mu$m, or a $d_{10}$ of at least about 80 $\mu$m and a $d_{90}$ of no greater than about 350 $\mu$m, or a $d_{10}$ of at least about 80 $\mu$m and a $d_{90}$ of no greater than about 300 $\mu$m. In such embodiments, the perlite microsphere may have a density of from about 0.20 to about 1.00 g/cc, for example, from about 0.30 g/cc to about 0.70 g/cc, or from about, or from about 0.35 g/cc to about 0.65 g/cc. Said perlite microsphere may have a $d_{50}$ of from about 150 $\mu$m to about 300 $\mu$m, for example, from about 150 $\mu$m to about

200 $\mu$m, or from about 200 $\mu$m to about 300 $\mu$m, or from about 225 $\mu$m to about 275 $\mu$m, or from about 240 $\mu$m to about 270 $\mu$m.

In certain embodiments, the perlite microsphere has a $d_{90}$ of no greater than about 900 $\mu$m, for example, no greater than about 700 $\mu$m, or no greater than about 600 $\mu$m, or no greater than about 500 $\mu$m, or no greater than about 475 $\mu$m, or no greater than about 450 $\mu$m, or no greater than about 425 $\mu$m, or no greater than about 400 $\mu$m, or no greater than about 375 $\mu$m, or no greater than about 350 $\mu$m, or no greater than about 325 $\mu$m, or no greater than about 300 $\mu$m, or no greater than about 275 $\mu$m, or no greater than about 250 $\mu$m, or no greater than about 225 $\mu$m, or no greater than about 200 $\mu$m, no greater than about 175 $\mu$m, or no greater than about 150 $\mu$m, or no greater than about 125 $\mu$m, no greater than about 100 $\mu$m. In certain embodiments, the perlite microsphere has a $d_{90}$ of no greater than about 90 $\mu$m, for example, no greater than about 80 $\mu$m, or no greater than about 70 $\mu$m, or no greater than about 60 $\mu$m, or no greater than about 50 $\mu$m. In certain embodiments, the perlite microsphere has a $d_{90}$ of from about 10 $\mu$m to about 100 $\mu$m, for example, or from about 15 $\mu$m to about 100 $\mu$m, or from about 20 $\mu$m to about 100 $\mu$m, or from about 25 $\mu$m to about 100 $\mu$m, or from about 30 $\mu$m to about 100 $\mu$m or from about 35 $\mu$m to about 100 $\mu$m. In certain embodiments, the perlite microsphere has a $d_{50}$ of from about 0.1 $\mu$m to about 200 $\mu$m. In certain embodiments, the perlite microsphere has a $d_{50}$ of greater than about 10 $\mu$m, for example, equal to or greater than about 25 $\mu$m. In certain embodiments, the perlite microsphere has a $d_{50}$ of from about 0.5 $\mu$m to about 150 $\mu$m, for example, from about 1 $\mu$m to about 125 $\mu$m, or from about 2 $\mu$m to about 125 $\mu$m, or from about 3 $\mu$m to about 125 $\mu$m, or from about 5 $\mu$m to about 125 $\mu$m, or from about 10 $\mu$m to about 125 $\mu$m, or from about 10 $\mu$m to about 100 $\mu$m, or from about 10 $\mu$m to about 90 $\mu$m, or from about 10 $\mu$m to about 80 $\mu$m, or from about 10 $\mu$m to about 70 $\mu$m, or from about 15 $\mu$m to about 70 $\mu$m, or from about 20 $\mu$m to about 60 $\mu$m, or from about 20 $\mu$m to about 50 $\mu$m, or from about 20 $\mu$m to about 45 $\mu$m, or from about 20 $\mu$m to about 40 $\mu$m. In certain embodiments, the perlite microsphere has a $d_{50}$ of from about 0.1 $\mu$m to about 30 $\mu$m, for example, from about 0.5 $\mu$m to about 30 $\mu$m, or from about 1 $\mu$m to about 30 $\mu$m, or from about 2 $\mu$m to about 30 $\mu$m, or from about 1 $\mu$m to about 25 $\mu$m, or from about 1 $\mu$m to about 20 $\mu$m, or from about 1 $\mu$m or 2 $\mu$m to about 15 $\mu$m, or from about 1 $\mu$m or 2 $\mu$m to about 10 $\mu$m, or from about 5 $\mu$m to about 15 $\mu$m.

In certain embodiments, the perlite microsphere has a $d_{100}$ of no greater than about 700 $\mu$m, for example no greater than about 500 $\mu$m. In certain embodiments, the perlite microsphere has a $d_0$ of at least about 1 $\mu$m, or at least about 5 $\mu$m, or at least about 10 $\mu$m, or at least about 25 $\mu$m.

Any particular particle size distribution may be obtained using conventional methods known in the art, e.g., by screening. For example, screening may be carried out using an Alpine A-200 jet sieve, supplied by Hosakawa Alpine, Germany, with screens provided by Haver & Bocker. The screen apertures may be selected depending on the particle size distribution required. For example, screens with apertures of 100 $\mu$m and 500 $\mu$m may be used, particularly if it is desired to remove or significantly reduce oversized particles and undersized particles.

In certain embodiments, the perlite microsphere has a density of from about 0.10 to about 4.0 g/cc, for example, from about 0.10 to about 3.8 g/cc, or from about 0.10 to about 3.5 g/cc, or from about 0.10 to about 3.2 g/cc, or from about 0.10 to about 3.0 g/cc, or from about 0.10 to about 2.5 g/cc, or from about 0.10 to about 2.0 g/cc, or from about 0.10 to about 1.9 g/cc, or from about 0.10 to about 1.8 g/cc, or from about 0.10 to about 1.7 g/cc, or from about 0.10 to about 1.6 g/cc, or from about 0.10 to about 1.5 g/cc, or from about 0.10 to about 1.4 g/cc, or from about 0.10 to about 1.3 g/cc, or from about 0.10 to about 1.2 g/cc, or from about 0.10 to about 1.1 g/cc, or from about 0.10 to about 1.0 g/cc, or from about 0.10 to about 0.9 g/cc, or from about 0.10 to about 0.8 g/cc, or from about 0.10 to about 0.7 g/cc, or from about 0.10 to about 0.6 g/cc, or from about 0.10 to about 0.5 g/cc, or from about 0.10 to about 0.4 g/cc, or from about 0.20 to about 0.6 g/cc, or from about 0.20 to about 0.5 g/cc, or from about 0.20 to about 0.4 g/cc, or from about 0.25 to about 0.4 g/cc, or from about 0.30 to about 0.4 g/cc.

In certain embodiments, the perlite microsphere has a crush strength of from about 350 KPa to about 5500 KPa. Crush strength is a measure of the pressure required to crush a bed of perlite microspheres, held within a steel die set, by 30 % of its original volume. Details of the method which may be used to determine crush strength are appended to the detailed description. In certain embodiments, the perlite microsphere has a crush strength of greater than about 1000 KPa, or greater than about 2000, KPa, or greater than about 2500 KPa, or greater than about 3000 KPa, or greater than about 3500 KPa, or greater than about 4000 KPa, or greater than about 4500 KPa, or greater than about 5000 KPa. In certain embodiments, the perlite microsphere has a crush strength of no greater than about 5250 KPa, or no greater than about 5000 KPa. Without wishing to be bound by theory, it is believed that a higher crush strength may increase the exfoliation properties of the personal care composition.

In certain embodiments, the perlite microsphere has a bulk density of from about 150-500 g/l, for example, from about 200-500 g/l, or from about 250-500 g/l, or from about 300-500 g/l, or from about 350-500 g/l, or from about 400-500 g/l, or from about 450-500 g/l, or from about 150-450 g/l, or from about 150-400 g/l, or from about 150-350 g/l, or from about 150-300 g/l, or from about 150-250 g/l, or from about 150-200 g/l. As used herein, the 'bulk density' of a substance is the value obtained when the mass of the substance is divided by its contained volume, after the substance has been subjected to conditions of free pouring. Bulk density may be determined in accordance with the test method, details of which are appended to the detailed description.

*Other components*

In certain embodiments the perlite microsphere further comprises a binder. In certain embodiments the coating layer of the perlite microsphere further comprises a binder. In certain embodiments the binder adheres the coating layer to the surface of the perlite microsphere or to the surface of the undercoat. The binder may be selected from any species capable of acting as a binder in the perlite microsphere, for example, in the coating layer of colourant. In certain embodiments the binder is present in an amount from about 0.1 to about 5 wt. % (based on the total weight of the perlite microsphere starting material), for example, from about 0.3 to about 4 wt. % (based on the total weight of the perlite microsphere), or from about 0.4 to about 3 wt. %, or from about 0.5 to about 2 wt. %. In certain embodiments the binder is mixed with the coating layer prior to addition to the perlite microsphere. In certain embodiments the binder is first dispersed in water before being mixed with the coating layer.

*Preparative methods*

The perlite microsphere having a modified visual appearance may be prepared by any suitable method. In certain embodiments, the method comprises treating a perlite microsphere such that its visual appearance is modified compared to the unmodified perlite microsphere prior to treatment. In certain embodiments, the perlite microsphere is treated with a colourant, producing a coloured perlite microsphere, as described above.
In certain embodiments, treating comprises:

(i) burnishing the perlite microsphere in the presence of a colourant, optionally wherein the perlite microsphere is coated with an undercoat prior to burnishing; or
(ii) mixing the perlite microsphere and colourant or colourant precursor in solution; or
(iii) vibrating a mixture of the perlite microsphere and colourant; or
(iv) spraying colourant or colourant precursor onto the surface of the perlite microsphere; or
(v) precipitating colourant onto the surface of the perlite microsphere; or
(vi) coating colourant on the perlite microsphere via a sol-gel process; or
(vii) forming an undercoat on the surface of the perlite microsphere followed by any one of (i)-(vi).

The undercoat may be a silver-based coating or a coating of silver.
In certain embodiments, the method further comprises one or more of washing, for example, with water and/or a surfactant solution, filtering and drying. The method may comprise multiple washing steps, for example, washing with water, followed by a surfactant wash, and further washing with water. Each step may itself comprise a number of sub-steps, for example, washing with water two or more times, followed by a surfactant wash, followed with a further two or more water washes. Suitable surfactants include non-ionic surfactants, for example, alkylphenol alkoxylate, such as nonylphenol ethoxylated. Suitable surfactants include the Tergitol™ range available from Dow, e.g., Tergitol™ NP-9. Suitable cationic, anionic and/or amphoteric surfactants may be used also.

*Compositions*

The perlite microspheres having a modified visual appearance, for example, an enhanced visual appearance may be utilised and incorporated in a variety of compositions. In certain embodiments, the composition is a personal care composition, for example, a personal care cleansing composition. In certain embodiments, the composition is a cosmetic. In other embodiments, the composition is a cleaning composition other than a personal care cleaning composition, for example, a house-hold cleaning product (e.g., surface cleaner).
Perlite microspheres having a modified visual appearance may be utilised to enhance the appearance and, thus, enhance user-appeal of the composition. Thus, the perlite microspheres may be used as visually enhanced exfoliating component in personal care, cosmetic or cleaning compositions owing, at least in part, to its advantageous scrub-feel properties and enhanced visual appearance.
In certain embodiments, the composition further comprises a cosmetically acceptable base.
In certain embodiments, the composition is a personal care cleansing composition for example, a gel, for example, a shower gel, optionally wherein the perlite microspheres provide a scrub feel, skin exfoliation, or both. In certain embodiments, the personal care cleansing composition is a hair shampoo, for example, an anti-dandruff shampoo, optionally wherein the perlite microspheres aid or provide exfoliation of the skin of the scalp.
In certain embodiments, the amount of perlite microspheres present in the composition, for example, personal care composition (e.g., personal care cleaning composition), cosmetic or cleaning composition is an amount of from about 0.1 wt. % to about 40 wt. %, based on the total weight of the personal care cleansing composition, for example, from about 0.1 wt. % to about 30 wt.%, or from about 0.1 wt. % to about 20 wt. %, or from about 0.1 wt. % to about 15 wt. %, or from about 0.1 wt. % to about 10 wt. %, or from about 0.1 wt. % to about 9.0 wt. %, or from about 0.2 wt. % to about 8.0 wt.%, or from about 0.3 wt. % to about 7.0 wt. %, or from about 0.4 wt.% to about 6.0 wt. %, or from about 0.5 wt. % to about 5.0 wt. %, or from about 0.5 wt.% to about 4.0 wt.%, or from about 0.5 wt.% to about 3.0 wt. %, or from about 0.5 wt. % to about 2.0 wt. %, or from about 0.75 wt. % to about 5.0 wt.%, or from about 0.75 wt. % to about 3.0 wt. %, or from about 0.75 wt. % to about 2.5 wt. %, or from about 1 wt. % about 3.0 wt. %, or from about 1.5 wt. % to about 5.0 wt. %, or from about 2.0 wt. % to about 10 wt. %, or from about 2.0 wt. % to about 5.0 wt. %, or from about 2.5 wt. % to about 5 wt. %, or from about 3.0 wt. % to about 10 wt. %, or from about 3.0 wt. % to about 8 wt. %.

The cosmetically acceptable base may be in the form a liquid, gel, emulsion, lotion or paste. In certain embodiments, the base is a gel. In certain embodiments, the base is a liquid. In certain embodiments, the cosmetically acceptable base comprises or constitutes the components of the composition other than the perlite microspheres.

Thus, the personal care, cosmetic or cleaning composition may contain one or more additional components suitable for the intended use.

For example, in certain embodiments, the personal care, cosmetic of cleaning composition comprises one or more surfactants. The one or more surfactants may constitute the detergent base of a gel. The one or more surfactants may be selected from zwitterionic, anionic, non-ionic and amphoteric surfactants, and mixtures thereof.

In certain embodiments, the surfactant(s) are present in a total amount ranging from about 1 wt. % to about 60 wt. %, based on the total weight of the composition, for example, from about 5 wt. % to about 50 wt. %, or from about 5 wt. % to about 30 wt. %. The skilled person will be able to select suitable amounts of surfactant for incorporation in the base, based on the amount of surfactant in the final composition and its intended use

In certain embodiments in which the personal care cleansing composition is a hair shampoo, the hair shampoo comprises one or more of sodium laureth sulfate, sodium $C_{14-16}$ olefin sulfonate, sodium lauryl sulfoacetate, sodium cocoyl isethionate, sodium methyl cocoyl taurate, cocoamidopropyl betaine, cocoamide MEA, and mixtures thereof.

In certain embodiments in which the hair shampoo is an anti-dandruff shampoo, the shampoo additionally comprises one or more additives, i.e., chemicals, for treating dandruff. In certain embodiments, the additive for treating dandruff, i.e., anti-dandruff chemical is one or more of zinc pyrithone, a corticosteroid, an imidazole antifungal agent such as, for example, ketoconazole, selenium sulfide, and a hydoxypiridone such as, for example, ciclopirox. In certain embodiments, the anti-dandruff chemical comprises or is zinc pyrithone. In certain embodiments, the anti-dandruff chemical comprises or is ketoconazole. The anti-dandruff chemical may be used in a suitable, e.g., effective, amount. Suitable amounts may range from about 0.1 wt. % to about 5 wt. %, based on the total weight of the hair shampoo, for example, from about 0.1 wt. % to about 3 wt.%, or from about 0.1 wt. % to about 2 wt. %. The skilled person will be able to select suitable amounts of anti-dandruff chemical(s) for incorporation in the base, based on the amount of anti-dandruff chemical(s) in the final composition.

In certain embodiments, the shampoo comprises conditioning (anti-static) surfactants to soothe the scalp after washing with the anti-dandruff shampoo. Exemplary conditioning surfactants are hydroxypropyltrimonium chloride and polyglycerol laurate.

The personal care or cosmetic composition may contain other components conventionally found in cosmetic applications for skin and hair, including, without limitation, skin conditioning/moisturising agents, hair conditioning/moisturising agents perfumes, fragrances, opacifiers, pearlescing agents, colourings, preservatives, chelating agents, humectants, herb and/or plant extracts, essential oils, proteins, pH adjusting agents, and anti-microbials. The total amount of other components may be present in amount of from about 0.1 to about 30 wt. %, based on the total weight of the personal care cleansing composition, for example, from about 0.1 wt. % to about 20 wt. %, or from about 0.1 wt. % to about 15 wt. %, or from about 0.5 wt. % to about 10 wt. %, or from about 1 wt. % to about 10 wt. %, or from about 1 wt. % about 5 wt. %. The skilled person will be able to select suitable amounts of each component for incorporation in the base, based on the amount of the component in the final composition.

The term "cleaning composition" used herein means a composition which is compatible with hard surfaces and/or tableware. In certain embodiments, the cleaning composition is a hard surface cleansing composition or tableware cleansing composition. It is an advantage of the compositions according to the present invention that they may be used to clean/cleanse inanimate surfaces made of a variety of materials like glazed and non-glazed ceramic tiles, enamel, stainless steel, Inox®, Formica®, vinyl, no-wax vinyl, linoleum, melamine, glass, plastics, Teflon®, painted surfaces and the like.

The term "hard surface cleansing composition" or "tableware cleansing composition" as used herein means a composition comprising a solid (e.g., a powder), or a liquid, such as a gel of water and bases (e.g., liquid soap). Liquid compositions include compositions having a water-like viscosity as well as thickened compositions, such as gels and pastes.

The compositions in accordance with certain embodiments of the present invention may be made by conventional methods of preparing personal care cleansing compositions, e.g., shower gels or anti-dandruff shampoos.

As used herein, the term "cosmetic composition" means a composition intended to be applied to the human body for beautifying, promoting attractiveness, or altering the appearance without affecting the body's structure or functions. In certain embodiments, the cosmetic composition is a decorative cosmetic. Cosmetics include concealers, foundations, and powders requiring high or full coverage and homogeneity, including corrective and camouflage products.

In certain embodiments, the cosmetic composition is a powder (e.g., pressed or loose), a liquid, a gel, a cream (e.g., a cream emulsion), a dispersion or an anhydrous stick. In certain embodiments, the cosmetic composition is a powder, for example, a pressed powder such as, but not limited to, a powdered cosmetic compact. In certain embodiments, the cosmetic composition is a makeup, for example, a face makeup including, but not limited to, primer, concealer, foundation, blush (also known as rouge or blusher), bronzer, eye shadow, mascara, lipstick, contour powder, face powder (often used to set a foundation), highlighter, eyeliner, or eyebrow applicator (e.g., pencil). Loose powders include body powder, for example, baby powder.

In certain embodiments, the cosmetic composition comprises from about 1 % by weight to about 95 % by weight of perlite microspheres having a modified visual appearance (i.e., based on the total weight of the cosmetic composition) for example, from about 20 % to about 90 % by weight, or from about 30 % to about 90 % by weight, or from about 40 % to about 90 % by weight, or from about 50 % to about 90 % by weight, or from about 60 % to about 90 % by weight, or from about 65 % to about 85 % by weight, or from about 70 % to about 85 % by weight, or from about 75 % to about 85 % by weight, or up to about 95 % by weight perlite microspheres, or up to about 90 % by weight perlite microspheres, or up to about 85 % by weight perlite microspheres, or up to about 80 % by weight perlite microspheres.

In certain embodiments, the cosmetic composition comprises colourant and/or binder and/or cosmetically acceptable base in addition to the perlite microspheres. In certain embodiments, the binder, when present, may be a constituent of the cosmetically acceptable base. In certain embodiments, the cosmetic composition comprises colourant and binder, in addition to the perlite microspheres.

When present, the cosmetically acceptable base may be any base suitable for the intended purpose. In certain embodiments, the base is an oil and/or wax containing material. The base and, thus, the cosmetic composition, may comprise other components such as humectants, preservative, emollient, fragrance and antioxidant.

*Measurement of optical properties*

*CIELAB*

In 1976 the CIELAB or CIE L*a*b colour scale was created. CIE publication 15.2(1986), section 4.2 contains details on this colour scale. This scale provides a standard, approximately uniform color scale enabling comparison of colour values.

According to this scale, differences between points plotted in the color space correspond to visual differences between the colors plotted. The CIELAB color space is organized in a cube form. The L* axis runs from the top to bottom. The maximum for L* is 100, which represents a perfect reflecting diffuser. The minimum for L* is zero, which represents black. The a* and b* axes have no specific numerical limits. Positive a* is red. Negative a* is green. Positive b* is yellow. Negative b* is blue. A diagram representing the CIELAB color space is shown in Figure 1.

The total color difference, $\Delta E$, may also be calculated. $\Delta E$ is a single value that takes into account the differences between the L*, a*, and b* of the sample and standard.

*- conditions for measurement*

Instrumental: BYK Spectroguide Sphere Gloss spectrophotometer in reflectance mode
Iluminant: D65
Standard Observer Function: 10 degree

*Hunter L, a, b*

Alternatively, the Hunter L,a,b colour scale may be used to asses colour. Hunter L, a and b values are calculated using a square root function of the CIE XYZ.

Hunter L,a,b values may be collected, for example, on a Datacolor CHECK® II Spectrophotometer (Datacolor, Lawrenceville, New Jersey).

L, a, and b values rank the whiteness, red/green, and blue/yellow values spectrophotometrically by measuring the reflection of light off of a colored sample. Values of L, a, and b may be considered independently from each other such that, for example, relatively small changes in one value (such as b) may be highly desirable even with relatively larger changes in another value (such as L). L is numbered between 0 and 100, with 0 being a completely black sample and 100 being a completely white sample. The a value is the red/green value which is a positive number for red samples (the more positive, the redder) and negative for green samples (the more negative, the greener). The b value is similar to the a but looks at the blue/yellow values of the material. The more positive or negative the number, the more yellow or blue, respectively.

Figure 1 is also representative of the Hunter L, a, b colour scale (i.e., one may change the axes to L, a, and b).

*Crush strength test method*

This test measures the pressure required to crush a bed of material, held within a steel die set, by 30 % of its original volume.

60 cm$^3$ of sample is measured and transferred into the cylindrical die. The die has an internal diameter of 50.65 and an internal height of 60.9 mm. The die is gently shaken on a flat surface for 10 seconds to 'pack' the material down in the die. A piston (having a diameter complimentary to the diameter of the die) is placed gently on top of the sample in the die.

The height of the piston protruding above the top of the die is measured with a digital micrometer and recorded, which enables the bed depth of the sample before compression to be calculated.

A tensometer is set up with a 10 kN load cell fitted with clamp holder but no clamp. The die set with sample and piston is then placed under the cross-head of the tensometer and the cross-head is driven down so it is close to the top of the piston position in a tensometer.

The pressure is monitored as the piston is gradually driven into the die. The measurement is monitored and data analysed using Qmat software. The pressure at 30 % volume compression is then obtained.

*Bulk density test method*

The bulk density of a substance is the value obtained when the mass of the substance is divided by its contained volume, after the substance has been subjected to conditions of free pouring.

The test may be carried out on powders and granular materials. The materials are usually tested without prior drying, providing the material pours freely. The condition of free pouring is defined by the height of fall and the contained volume. The grain size of the test material is limited by the diameter of the funnel stem (see apparatus below).

*Apparatus (ISO9001 compliant)*

   funnel, internal diameter 11 cm, stem diameter 1 cm, length 12 cm
   containing vessel; internal diameter 2.5 cm, depth 10 cm
   balance; capable of weighing 1000 g to 0.01 g
   retort stand, clamp and boss
   straight edge
   ruler to measure 7 cm

*Method*

   attach funnel to the retort stand
   tare the weight of the containing vessel
   place the containing vessel under the funnel
   adjust the clamp and boss so that the stem of the funnel is vertical and its end 7 cm above the top of the containing vessel
   pour the test material into the funnel until the containing vessel overflows
   level the test material across the top of the containing vessel with the straight edge
   weigh and record the net weight of the test material

*Expression of results*

   bulk density (BD) is expressed as g/cm$^3$ and is calculated as follows:

$$BD = W/V$$

where W is the net weight of the test material recorded, and V is the volume of the containing vessel.

For the avoidance of doubt, the present application is directed to the subject-matter described in the following numbered paragraphs:

1. Perlite microsphere having a modified visual appearance, for example, an enhanced visual appearance; and optionally

(a) a $-\Delta b^*$ or $-\Delta b$ compared to the unmodified perlite microsphere; or
(b) a $+\Delta b^*$ or $+\Delta b$ compared to the unmodified perlite microsphere; or
(c) a $-\Delta a^*$ or $-\Delta a$ compared to the unmodified perlite microsphere; or
(d) a $+\Delta a^*$ or $+\Delta a$ compared to the unmodified perlite microsphere;
(e) a $b^*$ or $b$ which is further from 0 than the unmodified perlite microsphere; or
(f) an $a^*$ or $a$ which is further from 0 than the unmodified perlite microsphere; or
(g) a colour density which is greater than the colour density of the unmodified perlite microsphere.

2. Perlite microsphere according to numbered paragraph 1, wherein the perlite microsphere is coloured.

3. Perlite microsphere according to numbered paragraph 1 or 2, wherein the perlite microsphere is coloured with a colourant.

4. Perlite microsphere according to numbered paragraph 3, wherein the colourant is a surface treatment.

5. Perlite microsphere according to numbered paragraph 3 or 4, wherein the colourant is a pigment, dye or colouring agent other than a pigment or dye.

6. Perlite microsphere according to any one of numbered paragraphs 3-5, wherein the colourant is present as a coating layer about the surface of the microsphere.

7. Perlite microsphere according to any one of numbered paragraphs 3-6, wherein the colourant comprises a metal, for example, a transition metal or a metal selected from any of the following: Co, Ag, Pb, Fe, Cr, Ca, Na or Cu.

8. Perlite microsphere according to any preceding numbered paragraph, further comprising a binder.

9. Perlite microsphere according to any preceding numbered paragraph comprising a first coating layer and second coating layer on the first coating layer, wherein either or both of the first and second coating layer comprise colourant.

10. Perlite microsphere according to any preceding numbered paragraph, further comprising an undercoat, for example, an undercoat of silver, biopolymer, and/or aminoalkoxysilane.

11. Perlite microsphere according to any preceding numbered paragraph, wherein the perlite microsphere has:

(i) a $d_{50}$ of from about 0.1 $\mu$m to about 400 $\mu$m, for example, from about 0.1 to about 200 $\mu$m; and/or
(ii) a $d_{90}$ of no greater than about 900 $\mu$m, for example, no greater than about 500 $\mu$m, for example, no greater than about 400 $\mu$m; and/or
(iii) a $d_{10}$ of at least about 30 $\mu$m and a $d_{90}$ of no greater than about 900 $\mu$m, for example, no greater than about 500 $\mu$m, for example, a $d_{10}$ of at least about 30 $\mu$m and a $d_{90}$ of no greater than about 400 $\mu$m; and/or
(iv) a density of from about 0.10 to about 4.0 g/cc; and/or
(v) a bulk density of from about 150-500 g/l; and/or
(vi) a crush strength of greater than about 1000 kPa.

12. Perlite microsphere according to any preceding numbered paragraph, wherein the perlite microsphere is substantially closed and hollow.

13. Perlite microsphere according to any preceding numbered paragraph having:

   (i) a b* or b of less than 0, for example, -2 or less, or -5 or less, or -10 or less, or -15 or less, or -20 or less; or
   (ii) an a* or a of greater than 0, for example, at least 2, or at least 4, or at least 6, or at least 8, or at least 10; or
   (iii) a b* or b of at least 0, for example, at least 2, or at least 4, or at least 6, or at least 8, or at least 10; or
   (iv) an a* or a of less than 0, for example, -1 or less, or -3 or less, or -5 or less.

14. Perlite microsphere according to any preceding numbered paragraph, wherein a* or a is positive and further from 0 compared to the unmodified perlite microsphere, optionally greater than about 5, and (i) b* or b is less than a, optionally closer to 0 compared to the unmodified perlite microsphere, or (ii) b* or b is positive and within 1 of a* or a.

15. Perlite microsphere according to any one of numbered paragraphs 1-13, wherein b* or b is -10 or less, and (i) a* or a is less than 0, optionally greater than about -5, for example, greater than about -2, or (ii) a* or a is no greater than about 4, for example, no greater than about 3, and optionally always positive.

16. Perlite microsphere according to any one of numbered paragraphs 1-13, wherein a* or a is less than 0, for example, -2 or less, optionally wherein b* or b is no greater than about 15 and optionally always positive.

17. Perlite microsphere according to any one of numbered paragraphs 1-13, wherein a* or a, and b* or b, are both positive integers, and (i) a* or a is greater than b* or b, or (ii) b* or b is greater than a* or a.

18. Perlite microsphere according to any preceding numbered paragraph, having a - ΔL* or -ΔL compared to the unmodified perlite microsphere, and/or wherein L* or L is less than the L* or L of the unmodified perlite microsphere, and/or wherein L* or L is from about 20 to about 70, for example, from about 30 to about 65, or less than about 50.

19. A method of making a perlite microsphere according to any one of numbered paragraphs 1-18, comprising treating a perlite microsphere such that its visual appearance is modified compared to the unmodified perlite microsphere prior to treatment.

20. A method according to numbered paragraph 19, wherein the unmodified perlite microsphere is treated with a colourant producing a coloured perlite microsphere.

21. A method according to numbered paragraph 19 or 20, wherein treating comprises:

   (i) burnishing the perlite microsphere in the presence of a colourant, optionally wherein the perlite microsphere is coated with an undercoat prior to burnishing; or
   (ii) mixing the perlite microsphere and colourant or colourant precursor in solution; or
   (iii) vibrating a mixture of the perlite microsphere and colourant; or
   (iv) spraying colourant or colourant precursor onto the surface of the perlite microsphere; or
   (v) precipitating colourant onto the surface of the perlite microsphere; or
   (vi) coating colourant on the perlite microsphere via a sol-gel process; or
   (vii) forming an undercoat on the surface of the perlite microsphere followed by any one of (i)-(vi).

22. The method of numbered paragraph 21, further comprising one or more of washing, for example, with water and/or a surfactant solution, filtering and drying.

23. A composition comprising perlite microspheres according to any one of numbered paragraphs 1-18.

24. Composition according to numbered paragraph 23, wherein the composition is a personal care composition, a cosmetic composition or a cleaning composition.

25. Composition according to numbered paragraph 23, wherein the composition is a personal care cleansing composition, for example, an exfoliating cleansing composition.

26. Personal care cleansing composition according to numbered paragraph 25, wherein the cleansing composition is a shower gel or bath gel, or wherein the personal care cleansing composition is a hair shampoo, for example, an anti-dandruff shampoo.

27. Composition according to numbered paragraph 24, wherein the composition is a cosmetic composition, for example, an exfoliating cosmetic composition.

28. Composition according to any one of numbered paragraphs 23-27, further comprising a cosmetically acceptable base.

29. A visually enhanced exfoliating agent comprising, or consisting essentially of, or consisting of, perlite microspheres according to any one of numbered paragraphs 1-18.

30. Use of perlite microspheres according to any one of numbered paragraphs 1-18 in a personal care, cosmetic or cleaning composition to visually modify the appearance, for example, to enhance user-appeal, of the composition.

31. Use of perlite microspheres according to any one of numbered paragraphs 1-18 as a visually enhanced exfoliating component in a personal care, cosmetic or cleaning composition.

32. Use of perlite microspheres according to any one of numbered paragraphs 1-18 in: (i) a personal care composition, for example, a personal care cleansing composition such as, for example, a gel, for example, a shower gel, optionally wherein the perlite microspheres provide a scrub feel, skin exfoliation, or both; or (ii) a hair shampoo, for example, an anti-dandruff shampoo, optionally wherein the perlite microspheres aid or provide exfoliation of the skin of the scalp.

33. Perlite microsphere according to any of numbered paragraphs 1-18, or use according to any one of numbered paragraphs 30-32, wherein the ∆E of the perlite microsphere is no greater than 2.5 following exposure to UV radiation for 24 hours, and/or no greater than 10 following exposure to UV radiation for over 750 hours, for example, no greater than 8, or no greater than 5.

## EXAMPLES

### Example 1

The unmodified starting material was perlite microspheres having a $d_{90}$ of 270 $\mu$m. Colour data was generated based on the Hunter Lab color scale, as described above.
Sample 1 was prepared by dissolving 0.5 g of cobalt chloride in 20 g of water. After mixing for 10 minutes, the solution was sprayed onto 100 g of perlite microspheres and mixed homogenously. The wet perlite microspheres were subsequently dried in an oven at 150 °C. Sample 1 was pink in colour. Sample 2 was prepared using the same method as Sample 1 except 2 g of cobalt chloride was used. Sample 2 was teal in colour. Colour data for samples 1 and 2 are given in Table 1.
A further sample, Sample 3, was made by dispersing 3 g of sodium silicate in 20 g of water to form a solution. 0.5 g of ultramarine blue powder was subsequently added to the solution. After mixing for 10 minutes, the solution was sprayed onto 100 g of perlite microspheres and mixed homogenously. The wet perlite microspheres were subsequently dried in an oven at 150 °C. A blue coloured perlite microsphere was produced with colour data given in Table 1.
A further sample, Sample 4, was made by mixing homogenously 100 g of perlite microspheres with 0.5 g of ultramarine blue powder. A blue coloured perlite microsphere was produced with colour data shown in Table 1.

**Table 1.**

| Sample | L | a | b |
|---|---|---|---|
| Expanded perlite microspheres starting material | 54.95 | 1.52 | 3.26 |
| Sample 1 | 48.59 | 3.97 | 1.95 |
| Sample 2 | 46.89 | -0.60 | -6.23 |
| Sample 3 | 43.56 | -1.51 | -14.37 |
| Sample 4 | 34.31 | -0.98 | -19.62 |

Example 2

The unmodified starting material was perlite microspheres having a $d_{90}$ of 400 $\mu$m. Samples were prepared on a 1 g scale.

Sample 5 was prepared by treating the perlite microspheres with copper phthalocyanine blue. Blue coloured perlite microspheres were produced.

Sample 6 was prepared by treating the perlite microsphere with ultramarine-sodium aluminiumsilicate/sulphur. Blue coloured perlite microsphere were produced.

Sample 7 was prepared by treating the perlite microspheres with chromium hydroxide. Green coloured perlite microspheres were produced.

Sample 8 was prepared by treating the perlite microspheres with 4.7 wt. % silver based on the weight of the perlite microsphere starting material. Silver coloured perlite microspheres were produced.

Sample 9 was prepared by initially coating the perlite microspheres with silver in accordance with Sample 8. The silver coated perlite microspheres were subsequently burnished in the presence of lithol rubine BK. Red coloured perlite microspheres were produced.

Following treatment with the colourant, the samples were washed with water three times, followed by washing with a surfactant (2.5g Tergitol NP-9 in 250 ml water), followed by three further washes with water. The samples were then filtered and air dried.

Color data for each samples is summarized in Table 2. This was determined in accordance with the CIELAB color scale.

Each of samples 5-9 were subjected to an Ultraviolet light test. The UV light test was carried out at a temperature of 45 °C for up to 773 hours. At selected time intervals (see Table 3 below), the L*a*b* data (CIELAB color sclae) of the samples were measured. ∆E values were also calculated to measure the difference in L*a*b* values between time intervals. The data are summarised in Table 3.

**Table 2.**

| Sample | L* | a* | b* |
|---|---|---|---|
| Sample 5 | 58.625 | 2.31 | -20.42 |
| Sample 6 | 57.33 | 2.185 | -19.77 |
| Sample 7 | 62.545 | -5.295 | 12.425 |
| Sample 8 | 60.68 | 0.755 | 8.455 |
| Sample 9 | 48.98 | 7.135 | 6.97 |

**Table 3.**

| | Time (hours) | Sample 5 | Sample 6 | Sample 7 | Sample 8 | Sample 9 |
|---|---|---|---|---|---|---|
| L* | 0 | 58.63 | 57.33 | 62.55 | 60.68 | 48.98 |
| | 24 | 57.33 | 56.44 | 61.09 | 59.36 | 47.91 |
| | 168 | 58.65 | 56.89 | 60.74 | 58.64 | 48.15 |
| | 336 | 58.99 | 57.13 | 60.44 | 57.36 | 48.87 |
| | 773 | 59.75 | 57.65 | 60.03 | 56.17 | 49.46 |
| a* | 0 | 2.31 | 2.185 | -5.295 | 0.755 | 7.135 |
| | 24 | 1.605 | 1.325 | -5.685 | 0.925 | 5.92 |
| | 168 | 1.365 | 1.755 | -4.99 | 0.99 | 7.32 |
| | 336 | 1.25 | 2.04 | -4.725 | 1.09 | 5.655 |
| | 773 | 2.395 | 2.58 | -4.73 | 1.145 | 4.115 |
| b* | 0 | -20.42 | -19.77 | 12.425 | 8.455 | 6.97 |
| | 24 | -19.4 | -18.83 | 11.94 | 8.31 | 5.845 |

(continued)

|  | Time (hours) | Sample 5 | Sample 6 | Sample 7 | Sample 8 | Sample 9 |
|---|---|---|---|---|---|---|
|  | *168* | -14.295 | -15.45 | 11.75 | 8.385 | 5.84 |
|  | *336* | -12.725 | -13.705 | 11.815 | 7.855 | 6.405 |
|  | *773* | -11.255 | -13.235 | 11.7 | 7.53 | 6.88 |
| $\Delta$**E** | *24* | 1.805 | 1.575 | 1.59 | 1.345 | 1.985 |
|  | *168* | 6.195 | 4.38 | 1.96 | 2.06 | 1.43 |
|  | *336* | 7.775 | 6.075 | 2.265 | 3.4 | 1.61 |
|  | *773* | 9.235 | 6.555 | 2.68 | 4.625 | 3.07 |

**Claims**

1. Perlite microsphere having a modified visual appearance, for example, an enhanced visual appearance; and optionally

   (a) a -$\Delta$b* or -$\Delta$b compared to the unmodified perlite microsphere; or
   (b) a +$\Delta$b* or +$\Delta$b compared to the unmodified perlite microsphere; or
   (c) a -$\Delta$a* or -$\Delta$a compared to the unmodified perlite microsphere; or
   (d) a +$\Delta$a* or +$\Delta$a compared to the unmodified perlite microsphere;
   (e) a b* or b which is further from 0 than the unmodified perlite microsphere; or
   (f) an a* or a which is further from 0 than the unmodified perlite microsphere; or
   (g) a colour density which is greater than the colour density of the unmodified perlite microsphere.

2. Perlite microsphere according to claim 1, wherein the perlite microsphere is coloured, optionally coloured with a colourant, further optionally wherein the colourant is a surface treatment.

3. Perlite microsphere according to claim 2, wherein the colourant is present as a coating layer about the surface of the microsphere.

4. Perlite microsphere according to claim 2 or 3, wherein the colourant comprises a metal, for example, a transition metal or a metal selected from any of the following: Co, Ag, Pb, Fe, Cr, Ca, Na or Cu.

5. Perlite microsphere according to any preceding claim, wherein the perlite microsphere has:

   (i) a $d_{50}$ of from about 0.1 $\mu$m to about 400 $\mu$m, for example, from about 0.1 to about 200 $\mu$m; and/or
   (ii) a $d_{90}$ of no greater than about 900 $\mu$m, for example, no greater than about 500 $\mu$m, for example, no greater than about 400 $\mu$m; and/or
   (iii) a $d_{10}$ of at least about 30 $\mu$m and a $d_{90}$ of no greater than about 900 $\mu$m, for example, no greater than about 500 $\mu$m, for example, a $d_{10}$ of at least about 30 $\mu$m and a $d_{90}$ of no greater than about 400 $\mu$m; and/or
   (iv) a density of from about 0.10 to about 4.0 g/cc; and/or
   (v) a bulk density of from about 150-500 g/l; and/or
   (vi) a crush strength of greater than about 1000 kPa.

6. Perlite microsphere according to any preceding claim having:

   (i) a b* or b of less than 0, for example, -2 or less, or -5 or less, or -10 or less, or -15 or less, or -20 or less; or
   (ii) an a* or a of greater than 0, for example, at least 2, or at least 4, or at least 6, or at least 8, or at least 10; or
   (iii) a b* or b of at least 0, for example, at least 2, or at least 4, or at least 6, or at least 8, or at least 10; or
   (iv) an a* or a of less than 0, for example, -1 or less, or -3 or less, or -5 or less.

7. Perlite microsphere according to any preceding claim, wherein a* or a is positive and further from 0 compared to the unmodified perlite microsphere, optionally greater than about 5, and (i) b* or b is less than a, optionally closer to 0 compared to the unmodified perlite microsphere, or (ii) b* or b is positive and within 1 of a* or a.

8. Perlite microsphere according to any one of claims 1-6, wherein b* or b is -10 or less, and (i) a* or a is less than 0, optionally greater than about -5, for example, greater than about -2, or (ii) a* or a is no greater than about 4, for example, no greater than about 3, and optionally always positive.

9. Perlite microsphere according to any one of claim 1-6, wherein a* or a is less than 0, for example, -2 or less, optionally wherein b* or b is no greater than about 15 and optionally always positive.

10. Perlite microsphere according to any preceding claim, having a $-\Delta L^*$ or $-\Delta L$ compared to the unmodified perlite microsphere, and/or wherein L* or L is less than the L* or L of the unmodified perlite microsphere, and/or wherein L* or L is from about 20 to about 70, for example, from about 30 to about 65, or less than about 50.

11. A method of making a perlite microsphere according to any one of claims 1-10, comprising treating a perlite microsphere such that its visual appearance is modified compared to the unmodified perlite microsphere prior to treatment, for example, by treating an unmodified perlite microsphere with a colourant, producing a coloured perlite microsphere, wherein treating comprises:

(i) burnishing the perlite microsphere in the presence of a colourant, optionally wherein the perlite microsphere is coated with an undercoat prior to burnishing; or
(ii) mixing the perlite microsphere and colourant or colourant precursor in solution; or
(iii) vibrating a mixture of the perlite microsphere and colourant; or
(iv) spraying colourant or colourant precursor onto the surface of the perlite microsphere; or
(v) precipitating colourant onto the surface of the perlite microsphere; or
(vi) coating colourant on the perlite microsphere via a sol-gel process; or
(vii) forming an undercoat on the surface of the perlite microsphere followed by any one of (i)-(vi).

12. A composition comprising perlite microspheres according to any one of claims 1-10.

13. Composition according to claim 12, wherein the composition is a personal care composition, a cosmetic composition or a cleaning composition.

14. Composition according to claim 12, wherein the composition is a personal care cleansing composition, for example, an exfoliating cleansing composition.

15. Personal care cleansing composition according to claim 14, wherein the cleansing composition is a shower gel or bath gel, or wherein the personal care cleansing composition is a hair shampoo, for example, an anti-dandruff shampoo.

## FIGURE 1

Figure showing White L* = 100, Black L* = 0, Green -a*, Yellow +b*, Red +a*, Blue -b*

Europäisches
Patentamt

European
Patent Office

Office européen
des brevets

# EUROPEAN SEARCH REPORT

Application Number

EP 16 30 5203

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/191324 A1 (OREAL [FR]) 4 December 2014 (2014-12-04) * page 1, lines 4-27 * * page 2, line 17 - page 9, line 11 * * page 23, lines 6-42 * * examples 1-4 * | 1-5, 11-15 | INV. A61K8/02 A61K8/19 C09C1/40 C09C1/30 |
| X | US 2013/081556 A1 (WANG BO [US] ET AL) 4 April 2013 (2013-04-04) * figure 1 * * paragraphs [0030] - [0065] * * examples 1-7 * | 1-12 | |
| X | US 2010/154680 A1 (FRIEDRICH HOLGER [ZA] ET AL) 24 June 2010 (2010-06-24) * paragraphs [0025], [0028], [0032], [0036], [0051] - [0053] * * examples 1-7 * * table 1 * | 1-12 | |
| Y | US 6 143 405 A (PALMGREN CHARLOTTE M [US]) 7 November 2000 (2000-11-07) * column 9, line 61 - column 15, line 31 * | 1-15 | TECHNICAL FIELDS SEARCHED (IPC) A61K C09C |
| Y | US 8 585 818 B1 (JONES STEVEN ALAN [US]) 19 November 2013 (2013-11-19) * column 3, line 1 - column 12 * * examples 1-8 * * tables 1,2 * | 1-15 | |

The present search report has been drawn up for all claims

| Place of search | Date of completion of the search | Examiner |
|---|---|---|
| The Hague | 22 July 2016 | Marino, Emanuela |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 16 30 5203

This annex lists the patent family members relating to the patent documents cited in the above-mentioned European search report.
The members are as contained in the European Patent Office EDP file on
The European Patent Office is in no way liable for these particulars which are merely given for the purpose of information.

22-07-2016

| Patent document cited in search report | | Publication date | Patent family member(s) | | Publication date |
|---|---|---|---|---|---|
| WO 2014191324 | A1 | 04-12-2014 | EP | 3004255 A1 | 13-04-2016 |
| | | | FR | 3006176 A1 | 05-12-2014 |
| | | | JP | 2016520104 A | 11-07-2016 |
| | | | US | 2016106642 A1 | 21-04-2016 |
| | | | WO | 2014191324 A1 | 04-12-2014 |
| US 2013081556 | A1 | 04-04-2013 | CN | 101678635 A | 24-03-2010 |
| | | | EP | 2129518 A1 | 09-12-2009 |
| | | | US | 2010104873 A1 | 29-04-2010 |
| | | | US | 2013081556 A1 | 04-04-2013 |
| | | | WO | 2008118827 A1 | 02-10-2008 |
| US 2010154680 | A1 | 24-06-2010 | AT | 545683 T | 15-03-2012 |
| | | | AU | 2009212776 A1 | 10-03-2011 |
| | | | BR | PI0904811 A2 | 03-11-2010 |
| | | | CA | 2675909 A1 | 01-03-2010 |
| | | | CN | 101665631 A | 10-03-2010 |
| | | | DE | 102008045121 A1 | 04-03-2010 |
| | | | DK | 2159266 T3 | 29-05-2012 |
| | | | EP | 2159266 A1 | 03-03-2010 |
| | | | ES | 2379855 T3 | 04-05-2012 |
| | | | JP | 2010059422 A | 18-03-2010 |
| | | | PL | 2159266 T3 | 31-07-2012 |
| | | | US | 2010154680 A1 | 24-06-2010 |
| US 6143405 | A | 07-11-2000 | AU | 4228199 A | 17-01-2000 |
| | | | BR | 9911659 A | 20-03-2001 |
| | | | CA | 2334361 A1 | 06-01-2000 |
| | | | DE | 69911027 D1 | 09-10-2003 |
| | | | DE | 69911027 T2 | 03-06-2004 |
| | | | EP | 1095380 A1 | 02-05-2001 |
| | | | JP | 2002519830 A | 02-07-2002 |
| | | | US | 6143405 A | 07-11-2000 |
| | | | WO | 0000988 A1 | 06-01-2000 |
| US 8585818 | B1 | 19-11-2013 | NONE | | |

EPO FORM P0459

For more details about this annex : see Official Journal of the European Patent Office, No. 12/82

**EP 3 207 917 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060075930 A

- WO 2013053635 A